# EUROPEAN PATENT APPLICATION

(11) **EP 1 543 831 A1**
(43) Date of publication of application: **22.06.2005**
(21) Application number: 03029117.3
(22) Date of filing: 18.12.2003
(51) Int. Cl.: A61K 31/197, A61K 9/08, A61K 9/00

(54) **Pregabalin composition**

(71) Applicant: Pfizer GmbH Arzneimittelwerk Gödecke, 79090 Freiburg (DE)
(72) Inventor: Schneider, Michael, Dr., 79211 Denzlingen (DE); Meyer-Wonnay, Hans Richard, 79312 Emmendingen (DE)
(74) Representative: Tesch, Rudolf, Dr.

(57) **Abstract**

The application comprises a stable liquid Pregabalin preparation that is adjusted to a stable pH-range below 6,5 and above 5,5, preferably below 6,2 and above 5,8. The liquid preparation of Pregabalin is preserved by a combination of methyl- and ethyl-parabene in a w/v ratio of 3:1 to 5:1. The preparation also comprises saccharin-sodium as a sweetening agent and flavouring agents lacking aldehyde- or keto-functions.

## Description

Pregabalin, [S-(+)-3-isobutyl-4-amino-butyric acid], is a potent orally active anticonvulsant. Oral liquid drug formulations of Pregabalin are preferred for the treatment of young children and elderly patients, since these patient groups require dosage forms which have a pleasant taste and are easy to swallow. It is also highly preferable that such formulations can be individually dosed.
A preservation is especially necessary if the container of a liquid formulation is to be accessed each time when single dosages must be taken. In such case each opening of the container includes additional contamination with germs. Multi-dosage oral liquid drug forms must therefore be sufficiently preserved by adding preservatives which keep the preparation sterile for a longer period of time after the container has been opened for the first application of the medicament. In the case of Pregabalin a stability period of at least 2 years is required.

For pediatric formulations it is further particularly preferred to avoid the use of cariogenic sugars as sweetening agents.
With attempts to comply with the above requirements it turned out that Pregabalin is extremely difficult to handle as an active pharmaceutical by the following reasons:
Pregabalin is highly soluble in water (32.1 mg/ml). Similar as the related drug Gabapentin, Pregabalin has a strong bitter taste, which in case of pediatric preparations raises almost insoluble problems. As it is known from Gabapentin which has also a γ-amino-acid structure, such compounds, under usual storage conditions and also in the presence of water, tend to form a cyclic lactam in an intramolecular reaction rather than to form a peptide chain in an intermolecular reaction. The cyclic lactam of Pregabalin (4-isobutyl-pyrrolidin-2-one) is an undesired side product. It is, therefore, important to prevent Pregabalin from forming its cyclic lactam structure. There are no general rules, how the undesired lactam-formation can be prevented, since it occurs not only under acid but also under basic conditions. However, in a series of investigations, it turned out surprisingly that for Pregabalin there exists a very narrow pH-range, where the formation of the lactam in a substantial amount can be avoided. This, in view of stability, acceptable pH range is between 5.5 and 6.5. As soon as the pH-value is higher or lower the lactam formation increases considerably. The minimum formation of the lactam was found in the range of pH 5.8 to pH 6.2. According to the present invention this range is therefore preferred. The desired pH-range may be adjusted to the above values by adding conventional buffer compositions e.g. citrate-buffers and optionally pharmaceutically acceptable acids or basic compounds.

In order to find a feasible composition enabling the preparation of a liquid syrup composition, first experiments have been carried out. However, since Pregabalin is less soluble in water than Gabapentin, it turned out to be difficult to find excipients and conditions that are in line with the above requirements. In order to obtain an appropriate concentration of the active compound the minimum content of Pregabalin in an oral syrup should be 10 mg/ml, preferably the content is above 15 mg/ml.

A first subject of the present invention is therefore an aqueous pharmaceutical preparation for oral administration comprising Pregabalin as an active agent which is dissolved in an aqueous liquid containing suitable adjuvants in dissolved or dispersed form, characterized in that the acidity of the pharmaceutical preparation is kept in a stable pH-range below 6,5 and above 5,5, preferably 5,8 to 6,2.

As mentioned above the antimicrobial preservation must be adequate, since the liquid is to be packed in a multiple-dose container. However, it turned out that this problem is as difficult as the lactam-formation. For making a sufficiently sterile syrup of Pregabalin as set out above the following prerequisites must be fulfilled by the preservative applied:
- The preserving efficacy must be satisfactory between pH 5.5 and 6.5.
- The preservative must not cause a pH-shift in the liquid preparation.
- The preservative must not have any detrimental effect on the flavor.
- The preservative must be suitable for peroral applications.
- The preservative must be sufficiently soluble in storage at 2°C to 8°C.
- The preservative must not interact chemically with the active compound.
- The preservative must not interact chemically with other excipients.

Besides the preservative the above criteria are of course also valid for other excipients that will be described in more detail below.

A number of possible preservatives turned out not to meet the above requirements:
A batch prepared with sorbic acid, one of the most common preservatives, in contact with Pregabalin showed not only discoloration but also an unacceptable pH-shift to 5.0 which strongly supports the formation of lactam. A preservation with glycerol did not comply with the requirements of the European Pharmacopeia. Ethanol is generally a critical preserving agent and must be completely avoided with pediatric formulations.
Following a series of negative tests it surprisingly turned out that methyl- and ethyl- paraben showed an acceptable compatibility with liquid Pregabalin formulations. The term liquid means that the given amount of the preservative [weight in mg(w)] is dissolved in a given volume [volume in ml (v)] of the liquid consisting of a solution or suspension of the active compound and any present adjuvant material.

However this is only true for a certain w/w-relationship of methyl- and ethyl-paraben. Outside a specific range, the activity was not sufficiently high versus certain strains of bacteria and fungi. For example, combinations of 1.20 mg methylparaben with 0.30 mg ethyl-paraben and 1.60 mg methyl-paraben with 0.40 mg ethyl-paraben per ml of the liquid did not meet the requirements of the European Pharmacopeia, because the required reduction of CFU for Candida albicans and Aspergillus niger was insufficient. Surprisingly a distinct w/v-ratio in the combination of methylparaben with ethylparaben met the requirements of European Pharmacopeia and was found to be especially useful for liquid Pregabalin preparations and had surprisingly no substantial influence to the critical parameters set out above.
According to the invention the w/w-ratio of methyl-paraben to ethyl-paraben should be 3 : 1 to 5 : 1. Preferred is a range of 3,5 - 4,5 : 1. The most preferred combination is 2 mg methyl-paraben with 0.5 mg ethyl-paraben per ml liquid.

A further subject of the present invention is therefore an aqueous pharmaceutical preparation for oral administration comprising Pregabalin as an active agent which is dissolved in an aqueous liquid containing suitable adjuvants in dissolved or dispersed form which comprisesa parabene preservative as an adjuvant, preferably a methyl/ ethyl-parabene mixture wherein the w/w-relationship of methyl-paraben to ethyl-paraben is 3 : 1 to 5 : 1.

Since no cariogenic excipients should be used as sweetening agents, particularly for pediatric oral formulations, the compatability of xylitol was tested. However, preparations using xylitol as one of the most common non-cariogenic sweeteners in pharmaceutical preparations showed an unexpected precipitation reaction under storage conditions at 5 °C. Tests with other sweetening agents as e.g. glycerol failed as well. After a series of unsuccessful tests it was found that saccharin-sodium was compatible with the Pregabalin formulation under all of the above conditions.

A further subject of the present invention is therefore an aqueous pharmaceutical preparation for oral administration comprising Pregabalin as an active agent which is dissolved in an aqueous liquid containing suitable adjuvants in dissolved or dispersed form which comprises saccharin-sodium as a sweetening adjuvant in a w/v (mg/ml) concentration of 2 : 1 to 1 : 2.

In addition to the sweetener, a suitable flavor is an essential instrument for masking the unpleasant taste. Tests with different sweeteners showed unexpectedly that in many cases undesired interactions between the flavor preparation and the content of the liquid preparation of Pregabalin occurred. Investigations demonstrated that aldehyde and keto- groups formed undesired side products which made them unsuitable for liquid Pregabalin preparations. According to the present invention care should be taken, that the used flavor preparations do not contain chemically active groups especially carbonyl-functions that may interfere with the active compound.
The following two flavors due to a lack of active keto- or aldehyde- functions showed the best results:
Strawberry Flavor 207420 (Haarmann & Reimer) and
Orange Flavor 9/055600 (Dragoco).

A further subject of the present invention is therefore an aqueous pharmaceutical preparation for oral administration comprising Pregabalin as an active agent which is dissolved in an aqueous liquid containing suitable adjuvants in dissolved or dispersed form which comprises a flavoring agent which is characterized by the absence of aldehyde- or keto-functions in the components of the flavoring agent.

In order to obtain a pharmaceutical liquid preparation of Pregabalin it is also advantageous to rise its viscosity because this measure does not only improve the handling when the liquid preparation is used but it has also been found surprisingly that the taste of the preparation can be greatly improved by just rising the viscosity. This may be done in the usual way by adding a suitable viscosity increasing agent such as hydroxyethylcellulose or xanthan gum. As set out above the primary amino-group present in the Pregabalin molecule not only is able to form a lactam-ring but also to react with other carbonyl functions. As discussed above for flavour compositions, which in many cases have aldehyde- or keto-functions, it is also advisable to avoid the incorporation of other excipients owning such undesired functions. In the present case the viscosity increasing agents hydroxyethylcellulose and/or xanthan gum turned out to be chemically neutral and especially suitable for their use with Pregabalin. They may be used in an amount of 2 to 5, mg, preferably about 3 mg/ml of the liquid preparation. The term liquid means that the given amount of the viscosity increasing agent or thickener [weight in mg (w)] is dissolved in a given volume [volume in ml (v)] of the liquid consisting of a solution of the active compound and any present other adjuvant or excipient material.

A further subject of the present invention is an aqueous pharmaceutical preparation for oral administration comprising Pregabalin as an active agent which is dissolved in an aqueous liquid containing suitable adjuvants in dissolved or dispersed form which comprises as viscosity increasing agents hydroxyethylcellulose and/or xanthan gum.

The syrup-like pharmaceutical compositions according to the invention can be filled into single-dose or multiple-dose containers. As a single dose container a double-sachet of coated aluminium foil containing two half-doses may be useful. The multiple dose container is of course more variable in the dose volume and can be provided with an appropriate dosage aid (measurement beaker, measurement pipette). As multiple dose containers glass or plastic bottles with childproof screw closure may be suggested.
The following Examples illustrate the invention in more detail, however, they may in no way be used to restrict the scope of the claims:

| Example | | |
|---|---|---|
| Components (mg/mL) | A (mg/ml) | B (mg/ml) |
| PREGABALIN | 15 | 15 |
| Methylparaben | 2 | 2 |
| Ethylparaben | 0.5 | 0.5 |
| Saccharin sodium | 1 | 1 |
| Hydroxyethylcellulose 250 | 3 | 3 |
| HHX | | |
| Strawberry flavor 207420 | 2 | -- |
| H&R | | |
| Orange flavor 9/055600 | -- | 1 |
| Dragoco | | |
| Purified water | ad 1 | ad 1 |

Pregabalin and also the flavor compositions are heat-sensitive. On the other hand parabens need hot water to obtain an acceptable dissolution profile. Hot water is also helpful for dispersing hydroxyethylcellulose in order to avoid lumps.
A Millipore ® membrane filter (cellulose acetate with a pore size of 10 µm) was used for filtration. Membrane filters using polytetrafluorene or other suitable polymer material may be used as well.

5 Liters of purified and sterile water is placed in a coolable and heatable reactor and then heated to 80 °C. 10 g Methyl-paraben and 2,5 g ethyl-paraben are stirred into the hot water. After a clear solution was obtained, 5 g saccharin sodium were added. In a further step 45 g hydroxyethylcellulose were added in small portions by stirring vigorously to avoid lumps. When the parabens and saccharin sodium were completely dissolved and the hydroxyethylcellulose was dispersed, the liquid was cooled to 30°C and 75g Pregabalin were added in dispersed, the liquid was cooled to 30°C and 75g Pregabalin were added in small portions by stirring. After the complete dissolution of Pregabalin cooling was continued and 10 g resp. 5 g of the flavor compositions were added at 25 °C to 30 °C. The flavour composition was dispersed homogeneously by stirring for at least 10 minutes. The solution was then freed of particulate impurities >10 µm by filtration, cooled to ambient temperature and stored in sealed containers.

## Claims

1. An aqueous pharmaceutical preparation for oral administration comprising Pregabalin as an active agent which is dissolved or dispersed in an aqueous liquid containing suitable adjuvants in dissolved or dispersed form, **characterized in that** the acidity of the pharmaceutical preparation is adjusted to a stable pH-range below 6,5 and above 5,5.

2. A pharmaceutical preparation according to Claim 1, wherein the pH range is in the range of 6,2 to 5,8.

3. A pharmaceutical preparation according to Claims 1 or 2, wherein the concentration of Pregabalin in the aqueous liquid is above 10 mg/ml.

4. A pharmaceutical preparation according to Claim 3, wherein the concentration is above 15 mg/ml.

5. A pharmaceutical composition according to anyone of Claims 1 to 4, comprising a parabene preservative as an adjuvant.

6. A pharmaceutical composition according to anyone of Claims 1 to 4, comprising methyl- and ethyl-parabene in a w/v (mg/ml) ratio of 3:1 to 5:1.

7. A pharmaceutical composition according to Claim 6, comprising saccharin-sodium as a sweetening agent in a w/v (mg/ml) ratio of 2:1 to 1:2.

8. A pharmaceutical composition according to anyone of Claims 1 to 7, wherein a flavour preparation is used as an adjuvant, which is **characterized by** the absence of aldehyde- and/or keto-functions in the components of the flavouring preparation.
